# EUROPEAN PATENT SPECIFICATION

(11) **EP 4 237 033 B1**
(45) Date of publication and mention of the grant of the patent: **07.08.2024**
(21) Application number: 21811527.7
(22) Date of filing: 28.10.2021
(51) Int. Cl.: A61M 1/16, B01D 63/02

(54) **OXYGENATOR OF ORGANIC FLUIDS**
OXYGENATOR FÜR ORGANISCHE FLÜSSIGKEITEN
OXYGÉNATEUR DE FLUIDES ORGANIQUES

(30) Priority: 29.10.2020 IT 202000025762
(43) Date of publication of application: 06.09.2023
(73) Proprietor: Spectrum Medical SRL, 41037 Mirandola (IT)
(72) Inventor: GALAVOTTI, Daniele, 41037 MIRANDOLA (IT)
(74) Representative: GCA S.R.L.
(86) International application number: PCT/IT2021/050357
(87) International publication number: WO 2022/091163

(56) References cited:
- WO-A1-00/06357
- WO-A1-2018/173092
- WO-A1-95/26488

## Description

### Field of the invention

The present invention concerns an oxygenator of organic fluids, usable in particular to oxygenate the blood flowing in an extracorporeal circuit.

### Background of the invention

For some time, oxygenating devices have been known, hereafter oxygenators for short, which are used to oxygenate an organic fluid, in particular blood, which flows in an extracorporeal circuit on which they are mounted and to which a patient is connected, for example subject to a cardiopulmonary support therapy. One example of these oxygenators of a type known in the state of the art is described in WO 00/06357. Another example is described in WO95/26488A1.

Typically, an oxygenator basically consists of a container that can have a cylindrical or parallelepiped shape, which internally defines a gaseous exchange chamber, or oxygenation chamber, in which a mass of hollow fibers is located.

The hollow fibers, which in practice are segments of capillaries, are disposed according to different criteria and have open lumens at the respective opposite ends. The fibers are made with a material that is porous to gases, but impermeable to liquids, so as to be able to be passed through internally only by a gas, in this specific case by oxygen, during the passage of the blood flow that laps the external surface of the fibers in a direction orthogonal to the longitudinal axes of the fibers.

The mass of fibers has all its ends open, which lead into respective chambers for the accumulation of oxygen to be delivered and for the accumulation of the carbon dioxide that is released from the blood during the gaseous exchange step.

The oxygen flows inside each fiber and is released into the blood flow during gas exchange, carrying out the oxygenation process. At the same time, the blood releases carbon dioxide which permeates through the membrane thickness of the hollow fibers which channels the exhausted gas toward the collection compartment from which it is expelled to the outside.

The container that forms the body of the oxygenator comprises at least two apertures for the entry of the oxygen and the exit of the carbon dioxide released in the gaseous exchange, and at least two other apertures for the entry of the blood to be treated and the exit of the blood treated.

The mass of hollow fibers is in turn retained by so-called "pottings" at the ends, that is, by monolithic elements made of polyurethane-based materials that incorporate their ends, blocking them in the fixed position inside the oxygenation chamber.

The mass of hollow fibers is typically made in the form of a bundle which is rolled up on a core or on itself in a direction transverse to the fibers, in such a way as to form a substantially cylindrical and elastically deformable body, in order to be adapted to the size and shape of the oxygenation chamber, defined by a cylindrical container mounted by interference on the rolled bundle. Other known solutions provide to fold a sheet of hollow fibers on itself, for example in bellows fashion, in which the hollow fibers are connected to each other by threads, generally of micrometric thickness, which determine a warp or weft. In any case, once installed, the hollow fibers are always in reciprocal contact with each other.

The disposition and installation of the hollow fibers is a critical aspect of oxygenators, since they are very expensive and very delicate to handle. During their installation there is in fact the risk that, if the fibers are pressed too hard, they will be crushed, causing the closure of one or more fibers, and also compromising the homogeneity of the hydraulic section passed through by the blood flow, consequently reducing the useful contact surface with the blood due to the excessive surface contact of capillary and capillary or layer and layer, or even more serious, they can be damaged and rupture causing a leakage of blood inside the cavities of the capillaries.

Document WO2018173092A1 discloses an oxygenator in which the mass of hollow fibers consists of a sheet of hollow fibers disposed parallel and folded in bellows fashion, and is equipped with one or more spacers located between two consecutive layers, so as to create a space between them with the purpose of allowing a correct outflow of blood. The spacers have flat elements with a central aperture that defines the hydraulic section useful for the outflow of blood. The oxygenator, in correspondence with a pre-chamber for the entry of the blood, also comprises dynamic distribution means, as well as two perforated septa to contain the mass of hollow fibers located upstream and downstream thereof, in relation to the direction of the blood outflow, which also perform a function of distributing the blood flow.

This solution does not fully satisfy the requirements of the sector, since the insertion of the spacers makes the step of assembling the mass of hollow fibers more complex and less fast. Furthermore, the disposition of the fibers is not perfectly controllable and faithfully reproducible according to the ideal hydraulic model, which can lead to irregularities in the network of fibers, entailing uneven blood flow.

Another disadvantage of using spacers is that it is impossible to reduce the overall sizes of the oxygenator, in particular the internal volume of the oxygenation chamber. In fact, the spacers themselves occupy a volume that cannot be reduced.

There is therefore a need to perfect an oxygenator which can overcome at least one of the disadvantages of the state of the art.

In particular, one purpose of the present invention is to provide an oxygenator which allows easier, faster and more controllable assembly of the mass of hollow fibers, and which at the same time guarantees its integrity.

Another purpose of the present invention is to provide an oxygenator which allows to reduce the risks of allergic reactions which blood can have in contact with extraneous surfaces.

Yet another purpose is to provide an oxygenator whose sizes and therefore the volume that has to be filled can be reduced compared with known oxygenators.

Yet another purpose is to provide an oxygenator whose total exchange surface of the capillaries in contact with the blood is as low as possible, given the same performances required, compared with known oxygenators.

Another purpose is to perfect a method to make a mass of capillary fibers which is easier, faster and more controllable than known methods.

The Applicant has devised, tested and embodied the present invention to overcome the shortcomings of the state of the art and to obtain these and other purposes and advantages.

### Summary of the invention

The present invention is set forth and characterized in the independent claims. The dependent claims describe other characteristics of the present invention or variants to the main inventive idea.

In accordance with the above purposes, an oxygenator of organic fluids is described below which overcomes the limits of the state of the art and eliminates the defects present therein.

In accordance with some embodiments, an oxygenator of organic fluids is provided comprising a container body inside which an oxygenation chamber is defined. The container body comprises a first aperture for the entry of a gas and a second aperture for the exit of an exhausted gas. Favorably, the gas is oxygen and the exhausted gas also contains carbon dioxide.

The container body also comprises a third aperture for the entry of an organic fluid, and a fourth aperture for the exit of the organic fluid. Advantageously, the organic fluid is blood. The third and fourth apertures are preferably located upstream and downstream of the oxygenation chamber with respect to the outflow of the organic fluid.

The oxygenator also comprises a plurality or mass of capillary fibers contained inside the oxygenation chamber. The capillary fibers are made of a material porous to gases and impermeable to liquids, so as to allow only the exchange of gas with the organic fluid to be treated. The capillary fibers are oriented parallel to each other in a first direction, and disposed in such a way as to be externally lapped by the organic fluid.

The mass of capillary fibers comprises at least two sheets of capillary fibers located in reciprocal contact along a respective surface thereof. In each sheet, the capillary fibers are connected and kept equidistant from each other by means of at least two connection threads oriented in a second direction. This second direction is orthogonal or inclined with respect to the first direction, in which the capillary fibers are oriented. Favorably, the connection threads are intertwined, or knotted, around each individual capillary fiber. The two sheets are disposed reciprocally offset in the first direction.

Preferably, each sheet of fibers is monolayer, that is, it comprises a single layer of capillary fibers.

Preferably, the two sheets are also offset in the second direction. In this way, the capillary fibers of each of the sheets are not located in correspondence with a capillary fiber of the other sheet, allowing the two sheets to be more homogeneously distanced.

In accordance with some embodiments, the oxygenator comprises a chamber for the entry of the organic fluid disposed between the third entry aperture of the organic fluid and the oxygenation chamber. Preferably, the entry chamber comprises static distribution means, configured to homogeneously distribute the flow of organic fluid.

In a preferred way, the oxygenator also comprises a chamber for the exit of the organic fluid located between the oxygenation chamber and the fourth exit aperture of the organic fluid. More preferably, the exit chamber comprises second static distribution means, configured to distribute the flow of blood homogeneously and direct it toward the fourth exit aperture of the organic fluid.

According to one aspect, there is also provided a method to form a mass of capillary fibers to be inserted in an oxygenation chamber of an oxygenator. The method provides to make available at least two sheets of capillary fibers each comprising a plurality of capillary fibers disposed parallel in a first direction, and at least two connection threads for connecting the capillary fibers, the connection threads being oriented in a second direction perpendicular, or inclined, with respect to the first direction. The at least two sheets are then disposed in reciprocal contact in correspondence with a respective face thereof, and offset in the first direction in such a way that the threads of one sheet are offset with respect to the threads of the other sheet. In this way, the threads that create the external weave on the various capillary fibers come into contact with the capillary fibers of the other sheet.

In accordance with some embodiments, the sheets are supplied by respective reels. Such reels are preferably disposed offset in the first direction.

In accordance with some embodiments, the method provides to dispose the sheets in such a way that the reciprocal contact between them occurs only between the threads of each sheet and the capillary fibers of the other sheet.

### Brief description of the drawings

These and other aspects, characteristics and advantages of the present invention will become apparent from the following description of some embodiments, given as a non-restrictive example with reference to the attached drawings wherein:
- fig. 1 is a perspective view of an oxygenator of organic fluids according to the embodiments described here;
- fig. 2 is a cross-section view of the oxygenator of fig. 1;
- fig. 3 is a bottom perspective view of a component of the oxygenator of fig. 1;
- figs. 4A and 4B are respectively a perspective view and a plan view of a second component of the oxygenator of fig. 1;
- fig. 5A is a schematic view of two reels of sheets of capillary fibers during a step of making a detail of the oxygenator;
- fig. 5B is an enlarged view of the detail A of fig. 5A;
- fig. 5C is a lateral view of a segment of the two sheets of capillary fibers of fig. 5B;
- fig. 6 is a section view of the oxygenator according to one variant; and
- fig. 7 is a perspective view of a step of making the oxygenator of fig. 6.

To facilitate comprehension, the same reference numbers have been used, where possible, to identify identical common elements in the drawings. It is understood that elements and characteristics of one embodiment can conveniently be combined or incorporated into other embodiments without further clarifications.

### Detailed description of a preferred example embodiment

We will now refer in detail to the possible embodiments of the invention, of which one or more examples are shown in the attached drawings, by way of a non-limiting example. The phraseology and terminology used here is also for the purposes of providing non-limiting examples.

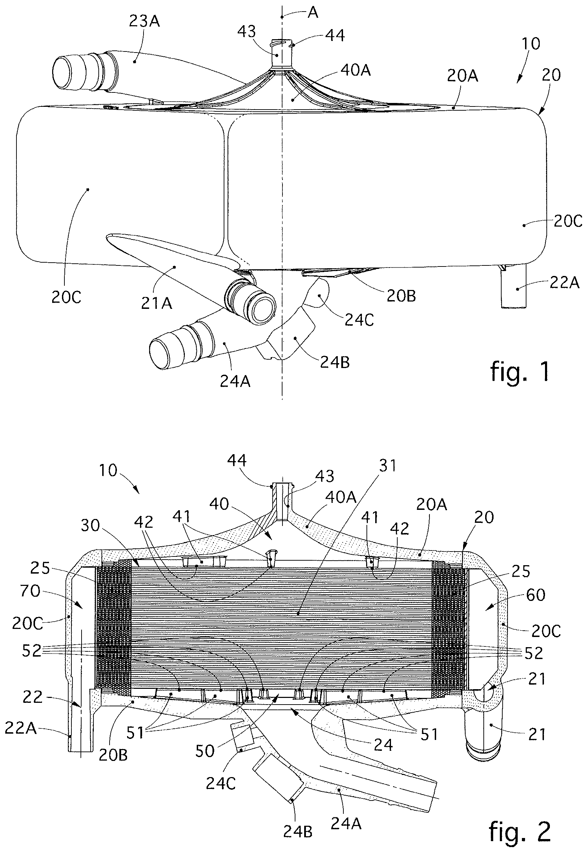

Fig. 1 shows an oxygenator of organic fluids, indicated as a whole with number 10. The oxygenator 10 comprises a container body 20 which delimits an oxygenation chamber 30 inside it (fig. 2).

Advantageously, the container body 20 defines a longitudinal axis A and is preferably parallelepiped in shape, that is, it comprises an upper wall 20A and a lower wall 20B which are opposite and perpendicular to the longitudinal axis A, as well as four lateral walls 20C connected to the upper and lower walls 20A, 20B, and preferably extended along their own axis parallel to the longitudinal axis A.

The container body 20 comprises a first aperture 21 for the entry of a gas, and a second aperture 22 for the exit of the same gas, in an exhausted condition. In the specific case of an oxygenator, the gas is oxygen, and the exhausted gas can comprise oxygen and contains carbon dioxide.

In the example shown, the first aperture 21 and the second aperture 22 are located in correspondence with two lateral walls 20C opposite each other, in such a way that the flow of gas passes through the oxygenation chamber 30 (fig. 2) from side to side. However, it is possible to provide other locations of the first and second apertures 21, 22, provided they are such as to determine a path of the gas that passes through the oxygenation chamber 30 at least once.

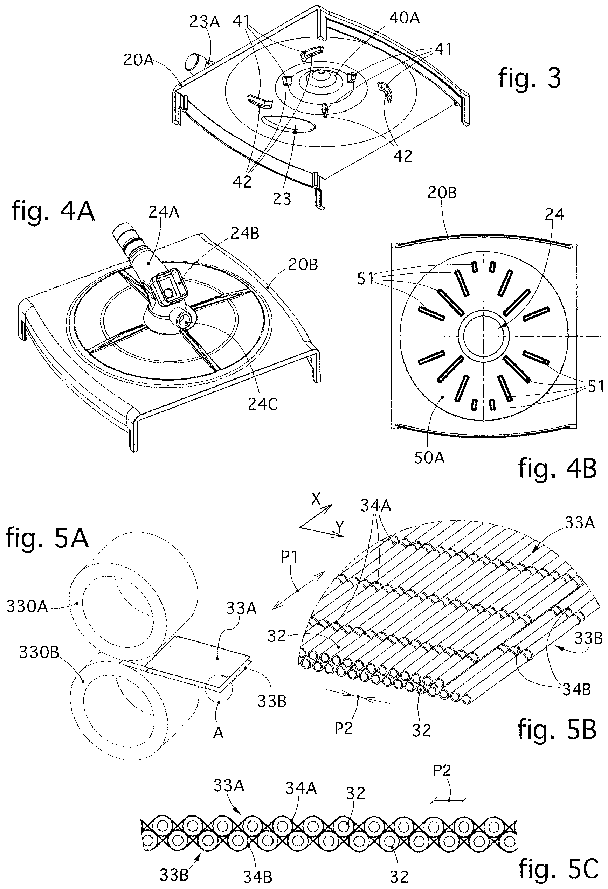

The container body 20 also comprises a third aperture 23 for the entry of the organic fluid and a fourth aperture 24 for the exit of the organic fluid (figs. 2 and 3). In this case, the organic fluid to be treated, that is, oxygenated, is blood, however the oxygenator is compatible with other types of organic fluids.

It should be noted that each of the apertures 21, 22, 23, 24 is provided with a corresponding connection duct 21A, 22A, 23A, 24A to allow the connection to corresponding systems for supplying gas or organic fluid (figs. 1, 2 and 3).

In an advantageous way, it can be provided that the connection duct 24A of the fourth aperture 24 is provided with one or more connection elements 24B, 24C (figs. 1, 2 and 4A) configured to allow the connection of instruments or accessories, such as for example devices for measuring parameters of the organic fluid.

It is preferable that the third aperture 23 and the fourth aperture 24 are positioned respectively upstream and downstream of the oxygenation chamber 30, with respect to the sense in which the organic fluid flows out, which is advantageously parallel or longitudinal to the longitudinal axis A.

Specifically, and in a preferential way, the third aperture 23 is positioned in the upper wall 20A of the container body 20, and the fourth aperture 24 is positioned in the lower wall 20B (figs. 1 and 2). In this way, the organic fluid flows from top to bottom through the oxygenation chamber 30.

It can be observed that the fourth aperture 24 is preferably in a central position with respect to the lower wall 20B, that is, it is centered with respect to the longitudinal axis A (figs. 2, 4A and 4B). The third aperture 23 is instead preferably distanced from the longitudinal axis A, and in any case in the upper wall 20A, more preferably in the dome 40A (fig. 3).

The third aperture 23 opens into the container body 20, advantageously into an entry chamber 40 located between the third aperture 23 and the oxygenation chamber 30. In the example shown in the drawings, the entry chamber 40 is made in the upper wall 20A of the container body 20, which forms a dome 40A extending toward the outside of the container body 20. Preferably, the dome is coaxial with respect to the container body 20, that is, their longitudinal axes coincide.

Favorably, the entry chamber 40 is equipped with static distribution means 41. In accordance with some embodiments, the distribution means 41 are made in a single piece in the upper wall 20A of the container body 20 and protrude from its internal surface (fig. 3). These distribution means 41 can be in the shape of fins, preferably curved with the concavity oriented toward the inside of the dome 40A.

More advantageously, the fins 41 are configured to have a flat lower surface 42, so that the flat lower surfaces 42 of all the fins 41 are disposed on the same plane, which delimits at the lower part the entry chamber 40 of the organic fluid (fig. 2). Preferably, the lower surface 42 of the fins 41 is perpendicular to the longitudinal axis A.

The dome 40A allows to accumulate air potentially present in the organic fluid, in such a way as to prevent the air from accumulating in the oxygenation chamber 30 and possible emboli from being created in the event that the organic fluid is blood. To allow the evacuation of the air, it is possible to provide an upper aperture 43 at the top of the dome 40A, preferably of a tubular shape extending toward the outside of the container body 20, and possibly equipped with connection means 44 so as to connect it to an external system (figs. 1 and 2).

Similarly, the lower wall 20B preferentially defines an exit chamber 50 of the organic fluid, located between the oxygenation chamber 30 and the fourth aperture 24 (fig. 2). This exit chamber 50 is suitably equipped with its own static distribution means 51, which can be configured as fins that protrude from the internal surface of the lower wall 20B. Advantageously, the fins 51 are made in a single piece in the lower wall 20B.

Favorably, the fins 51 are straight and oriented radially with respect to the fourth aperture 24, that is, with respect to the longitudinal axis A (fig. 4B). In this way, the outflow of the organic fluid at exit from the oxygenator 10 is improved. The fins 51 can have different lengths with respect each other, depending on their positioning, in particular depending on their distance from the fourth aperture 24.

The fins 51 have a flat upper surface 52 preferably oriented perpendicular to the longitudinal axis A. Preferably, the fins 51 are configured in such a way that their upper surfaces 52 are disposed on a same plane, advantageously perpendicular to the longitudinal axis A, which delimits the exit chamber 50 at the top.

It can be surmised that the oxygenation chamber 30 is contained between the lower surfaces 42 of the fins 41 of the upper wall 20A and the upper surfaces 52 of the fins 52 of the lower wall 20B (fig. 2). These surfaces 42, 52 of the fins 41, 51 combine to form an upper and lower rest plane for a mass 31 of capillary fibers 32 to be inserted into the oxygenation chamber. In this way, it is no longer necessary to add the perforated distribution plates present in the state of the art.

It should be noted that the lower surface 20B can define an internal zone 50A with a shape and sizes equal to those of the dome 40A (figs. 3 and 4B), in such a way as to define a useful hydraulic section in which the organic fluid is made to flow through the oxygenation chamber 30. The dome 40A and the internal zone 50A peripherally define the entry chamber 40 and the exit chamber 50 of the organic fluid, respectively.

Inside the oxygenation chamber 30 there is disposed a mass 31 of capillary fibers 32 made of a material porous to gases and impermeable to liquids. The capillary fibers 32 are all oriented in a same first direction X, preferably perpendicular to the longitudinal axis A (fig. 2). The capillary fibers 32 are open at both their ends, in such a way as to allow the entry and exit of gas from inside them. The open ends of the fibers can thus be isolated from the oxygenation chamber 30 to prevent the organic fluid from obstructing them.

For this purpose, the oxygenator 10 can comprise a support element 25 to support the capillary fibers 32 which is conformed to only engage their ends (fig. 2). This support element 25 is known by the name "potting". Advantageously, the "potting" is configured in such a way as to not interfere, or only marginally interfere, with the useful hydraulic section of the oxygenator 10.

The capillary fibers 32 thus disposed put in fluidic communication a first lateral chamber 60, located laterally with respect to the oxygenation chamber 30, and a second lateral chamber 70, also located laterally with respect to the oxygenation chamber 30, but on the opposite side to the first lateral chamber 60.

Advantageously, the first lateral chamber 60 is delimited externally by the lateral wall 20C where the first aperture 21 is located, while the second lateral chamber 70 is delimited externally by the lateral wall 20C where the second aperture 22 is located (fig. 2). In other words, the first lateral chamber 60 is located between the first aperture 21 and the oxygenation chamber 30, and the second lateral chamber 70 is located between the second aperture 22 and the oxygenation chamber 30.

This configuration of the lateral chambers 60, 70 can be achieved by providing that they are made in two respective half-bodies which are able to be reciprocally coupled in a hermetic way and which combine to form the container body 20. The half-bodies can for example have a substantially semi-annular shape, so as to delimit the hydraulic section when they are coupled to each other.

The mass of fibers 31 is obtained by using two sheets 33A, 33B of capillary fibers 32. Each sheet 33A, 33B consist of a plurality of capillary fibers 32 disposed parallel to each other in a first direction X, and connected to each other by means of at least two threads 34A, 34B intertwined on the external surface of the capillary fibers 32 and oriented in a second direction Y inclined with respect to the first direction X, preferably perpendicular to the first direction X (figs. 5B and 5C). The second direction Y is also perpendicular to the longitudinal axis A. The threads 34A, 34B are useful for also keeping the capillary fibers 32 equidistant from each other.

It should be noted that in each sheet 33A, 33B the capillary fibers 32 preferentially form a single layer. The capillary fibers 32 can have a diameter of the order of a few hundred microns, for example 380µm, and the threads 34A, 34B can have a thickness of the order of 10µm.

Each sheet 33A, 33B preferably comprises a plurality of threads 34A, 34B which are regularly distributed along the length of the capillary fibers 32 according to a predefined pitch P1. The pitch P1 can be of the order of a few millimeters, for example 10mm.

The two sheets 33A, 33B are placed in reciprocal contact along a respective surface defined by the capillary fibers 32 (fig. 5B); pratically, the capillary fibers 32 are never in contact with each other because the contact occurs between the threads 34A, 34B of one sheet 33A, 33B, which protrude by their thickness on the external surface of the capillaries, and the capillary fibers 32 of the other sheet 33A, 33B. In this way, they form a sheet with a double layer of capillary fibers 32. The sheets 33A, 33B are disposed so that the threads 34A of a first sheet 33A are offset in the first direction X with respect to the threads 34B of the second sheet 33B. Obviously, the offset is preferably not equal to the pitch P between the threads 34A, 34B of a same sheet.

In this way, the contact between the two sheets 33A, 33B only actually occurs between the threads 34A, 34B of one of the sheets and the fibers 32 of the other sheet. Furthermore, instead of having a predetermined pitch, for example of 10mm between the threads 34A, 34B, there is obtained an alternation of threads 34A, 34B between the capillary fibers 32 of the two sheets, wherein the threads 34A, 34B are distanced by a smaller pitch, for example smaller than 10mm. This alternation of threads 34A, 34B creates a layer of threads which is interposed between the capillary fibers 32 of the two sheets 33A, 33B.

By doing so, the installation of the capillary fibers 32 in the oxygenation chamber 30 is easier, faster and more controllable than in the state of the art. The capillary fibers 32 are regularly distanced from each other, creating a homogeneous and repeatable three-dimensional matrix which defines a hydraulic section useful for the passage of the organic fluid such as to prevent the capillary fibers 32 from touching each other. Furthermore, a contact surface for the organic fluid is freed which, the number of capillary fibers 32 being equal, is higher and therefore allows to reduce the useful contact surface between the fibers and the organic fluid. In the case of blood, this is an advantage since it reduces the risk of allergic reactions of the blood with the material of the fibers.

All this also allows to reduce the sizes of the oxygenator 10, in particular of its internal volume.

One way of achieving the offset of the threads 34A, 34B is simply to offset the two sheets 33A, 33B in the first direction X. It can for example be provided to resort to two reels 330A, 330B of sheets 33A, 33B of capillary fibers 32, and dispose them reciprocally offset in the first direction X in such a way as to be able to supply the sheets already offset with respect to each other (fig. 5A).

The offset between the threads 34A, 34B of the two sheets 33A, 33B is advantageously smaller than the pitch P1 between the threads 34A, 34B of a same sheet 33A, 33B, so that the threads are separated from each other by a distance smaller than the pitch P1. Advantageously, the threads 34A, 34B are offset by a half pitch P1, in such a way as to be regularly distanced from each other by a distance equal to half the pitch P. In the case of a pitch P1 equal to 10mm, the threads 34A, 34B are distanced by 5mm in the sheet with a double layer (fig. 5C).

In accordance with some embodiments, the sheets 33A, 33B are offset with respect to each other also in the second direction Y.

In each sheet 33A, 33B, the capillary fibers 32 are suitably separated, in the second direction Y, by a second pitch P2. It is particularly advantageous to provide that the sheets 33A, 33B are offset by half of the second pitch P2, so that the capillary fibers 32 of a first sheet 33A are located between two capillary fibers 32 of the second sheet 33B (fig. 5C). In this way, the capillary fibers 32 form a more compact and more stable three-dimensional net.

In the embodiment shown in fig. 2, the mass 31 of capillary fibers 32 consists of two sheets 33A, 33B disposed as described above and folded in bellows fashion so as to fill the oxygenation chamber 30.

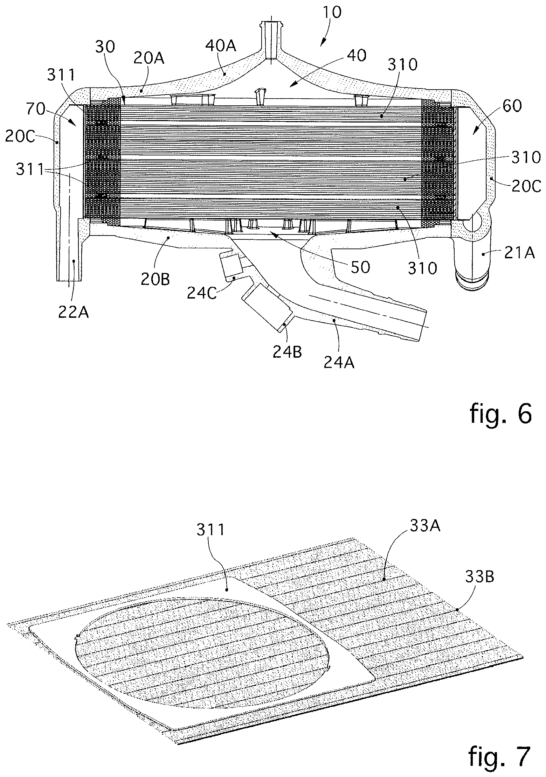

Fig. 6 shows an alternative embodiment, in which the capillary fibers 32 are conformed in sub-groups 310. Each sub-group 310 comprises a support element 311 around which two sheets 33A, 33B of capillary fibers 32, disposed as described above, are wound on several loops (fig. 7).

Favorably, the support element 311 has a substantially annular shape, with an internal aperture with a shape and sizes larger than those of the dome 40A, so as to not interfere with the useful hydraulic section of the oxygenator 10.

Below a mode of assembly of the oxygenator, and a functioning mode thereof are described.

It is first provided to make available the container body 20, of the type that can be dismantled or opened, so as to make the oxygenation chamber inside it accessible.

Subsequently, the mass 31 of capillary fibers 32 is prepared, by supplying two sheets 33A, 33B placed in reciprocal contact in correspondence with a surface thereof and disposed in such a way that the threads 34A of the first sheet 33A are offset with respect to the threads 34B of the second sheet 33B (fig. 5B). It can for example be provided that the two sheets 33A, 33B are supplied offset with respect to each other in the first direction X, by a distance preferably smaller than the pitch P between the threads 34A, 34B of a same sheet 33A, 33B.

Preferably, the two sheets 33A, 33B are fed by respective reels 330A, 330B, which can be disposed offset, in such a way that the sheets 33A, 33B are already offset when they are supplied.

It is advantageous to provide that the two sheets 33A, 33B are also offset in the second direction Y, so that each capillary fiber 32 of one sheet 33A is positioned resting on the respective threads between two capillary fibers 32 of the other sheet 33B (fig. 5C). The capillary fibers 32 of the two sheets 33A, 33B are therefore indicatively distanced from each other by 10 µm.

The two sheets 33A, 33B taken together (in such a way as to form a single sheet with a double layer of capillary fibers 32) are then shaped in order to enter the oxygenation chamber 30 in order to fill it. According to one embodiment, the sheets 33A, 33B are folded in bellows fashion, in order to obtain different double layers of capillary fibers 32 accumulated on each other. The mass 31 thus obtained is then inserted into the oxygenation chamber 30.

Alternatively, it can also be provided to prepare sub-groups 310 by preparing a support element 311 on a pair of sheets 33A, 33B, and to repeatedly wrap the latter around the support element 311 until a sub-group 310 with a predetermined thickness is obtained. The thickness can be any thickness whatsoever, according to the requirements or sizes of the oxygenation chamber 30 to be filled, or also the number of sub-groups 310 to be used.

The sub-groups 310 are then disposed stacked in the oxygenation chamber 30, as shown in fig. 6.

Once the mass 31 of capillary fibers 32 has been installed, the container body 20 is hermetically closed.

The oxygenator is subsequently connected, by means of the connection ducts 21A, 22A, 23A, 24A and possibly also by means of the upper aperture 43, if provided, to a suitable circuit, for example an extracorporeal circuit for blood circulation, or the circulation of another organic fluid.

The functioning of the oxygenator 10 is similar to that of known oxygenators 10.

It is clear that modifications and/or additions of parts or steps may be made to the oxygenator and to the method as described heretofore, without departing from the field and scope of the present invention as defined by the claims.

In the following claims, the sole purpose of the references in brackets is to facilitate reading: they must not be considered as restrictive factors with regard to the field of protection claimed in the specific claims.

## Claims

1. Oxygenator (10) of organic fluids, comprising
- a container body (20);
- a first aperture (21) for the entry of oxygen and a second aperture (22) for the exit of an exhausted gas, both obtained in said container body (20);
- a third aperture (23) for the entry of an organic fluid to be oxygenated and a fourth aperture (24) for the exit of an oxygenated organic fluid, both obtained in said container body (20);
- an oxygenation chamber (30) to oxygenate said organic fluid to be oxygenated, defined inside said container body (20);
- a mass (31) of capillary fibers (32) which are impermeable to liquids and porous to gases, disposed so as to be lapped by said organic fluid inside said oxygenation chamber (30), parallel to each other in a first direction (X);
wherein said mass (31) of capillary fibers (32) comprises at least two sheets (33A, 33B) of capillary fibers (32) connected to each other by means of at least two connection threads (34A, 34B) which extend in a second direction (Y) perpendicular to said first direction (X), said sheets 25 (33A, 33B) being in reciprocal contact along a respective surface thereof, said oxygenator (10) being **characterized in that** said sheets (33A, 33B) are arranged in such a way that the threads (34A) of a first sheet (33A) are offset with respect to the threads (34B) of the other sheet (33B) in said first direction (X).

2. Oxygenator (10) as in claim 1, **characterized in that** the two sheets (33A, 33B) are offset with respect to each other in the first direction (X).

3. Oxygenator (10) as in claim 1 or 2, **characterized in that** the two sheets (33A, 33B) of capillary fibers (32) are offset also in the second direction (Y).

4. Oxygenator (10) as in any claim hereinbefore, **characterized in that** the contact between the two sheets (33A, 33B) is made only between the threads (34A, 34B) of each sheet (33A, 33B) and the capillary fibers (32) of the other sheet (33A, 33B).

5. Oxygenator (10) as in any claim hereinbefore, **characterized in that** the connection threads (34A, 34B) of a same sheet (33A, 33B) are distanced by a predefined first pitch (P1), and that the two sheets (33A, 33B) are offset in the first direction (X) by a distance equal to half said first pitch (P1).

6. Oxygenator (10) as in claim 3, 4 or 5 if dependent on 3, **characterized in that** the capillary fibers (32) of a same sheet (33A, 33B) are distanced by a second pitch (P2), and that the two sheets (33A, 33B) are offset in the second direction (Y) by a distance equal to half said second pitch (P2).

7. Oxygenator (10) as in any claim hereinbefore, **characterized in that** it comprises a chamber (40) for the entry of the organic fluid to be treated, located between the third entry aperture (23) and the oxygenation chamber (30), and that said entry chamber (40) comprises static distribution means (41).

8. Oxygenator (10) as in claim 7, **characterized in that** the static distribution means (41) comprise fins preferably made in a single piece with an upper surface (20A) of the container body (20).

9. Oxygenator (10) as in claim 8, **characterized in that** the fins (41) have a flat lower surface (42), and that said flat lower surfaces (42) of all the fins (41) are disposed on the same plane.

10. Oxygenator as in any claim hereinbefore, **characterized in that** it comprises an exit chamber (50) of the organic fluid to be treated, located between the oxygenation chamber (30) and the fourth exit aperture (24), and that said exit chamber (50) comprises second static distribution means (51).

11. Oxygenator (10) as in claim 10, **characterized in that** the second static distribution means (51) comprise fins preferably made in a single piece with a lower surface (20B) of the container body (20).

12. Oxygenator (10) as in claim 11, **characterized in that** the fins (51) have a flat upper surface (52), and that said flat upper surfaces (52) of all the fins (51) are disposed on the same plane.

13. Method to form a mass (31) of capillary fibers (32) to be inserted in an oxygenation chamber (30) of an oxygenator (10), which provides to make available at least two sheets (33A, 33B) of capillary fibers (32), each of which comprises a plurality of capillary fibers (32) oriented parallel in a first direction (X), and at least two threads (34A, 34B) for connecting the capillary fibers (32), said two threads (34A, 34B) being oriented in a second direction (Y) perpendicular to said first direction (X), and to dispose said sheets (33A, 33B) in reciprocal contact along a respective surface thereof, wherein said sheets (33A, 33B) are disposed in such a way that the threads (34A) of a first sheet (33A) are offset with respect to the threads (34B) of the other sheet (33B) in said first direction (X).

14. Method as in claim 13, **characterized in that** the sheets (33A, 33B) are supplied by respective reels (330A, 330B) disposed offset in the first direction (X).

15. Method as in claim 13 or 14, **characterized in that** it is provided to dispose said sheets (33A, 33B) in such a way that the reciprocal contact is made only between the threads (34A, 34B) of each sheet (33A, 33B) and the capillary fibers (32) of the other sheet (33A, 33B).

## Patentansprüche

1. Oxygenator (10) für organische Fluide, der folgendes umfasst
- einen Behälterkörper (20);
- eine erste Öffnung (21) für den Eintritt von Sauerstoff und eine zweite Öffnung (22) für den Austritt eines Abgases, die beide in dem Behälterkörper (20) vorhanden sind;
- eine dritte Öffnung (23) für den Eintritt eines mit Sauerstoff anzureichernden organischen Fluids und eine vierte Öffnung (24) für den Austritt eines mit Sauerstoff angereicherten organischen Fluids, die beide in dem Behälterkörper (20) erhalten sind;
- eine Oxygenierungskammer (30) zur Anreicherung des organischen Fluids mit Sauerstoff, das mit Sauerstoff angereichert werden soll, die im Inneren des Behälters (20) definiert ist;
- eine Masse (31) von Kapillarfasern (32), die für Flüssigkeiten undurchlässig und porös für Gase sind, die so vorgesehen sind, dass sie von dem organischen Fluid im Inneren der Oxygenierungskammer (30), überlappend parallel zueinander in einer ersten Richtung (X) liegen;
wobei die Masse (31) von Kapillarfasern (32) mindestens zwei Lagen (33A, 33B) von Kapillarfasern (32) aufweist, die miteinander durch mindestens zwei Verbindungsfäden (34A, 34B) verbunden sind, die sich in einer zweiten Richtung (Y) senkrecht zur ersten Richtung (X) erstrecken, wobei die Lagen 25 (33A, 33B) in gegenseitiger Berührung entlang einer ihrer jeweiligen Oberflächen stehen, wobei der Oxygenator (10)
**dadurch gekennzeichnet, ist, dass** die Lagen (33A, 33B) so angeordnet sind, dass die Fäden (34A) einer ersten Lage (33A) in Bezug auf die Fäden (34B) der anderen Lage (33B) in der ersten Richtung (X) versetzt sind.

2. Oxygenator (10) nach Anspruch 1, **dadurch gekennzeichnet, dass** die beiden Lagen (33A, 33B) in der ersten Richtung (X) in Bezug zueinander versetzt sind.

3. Oxygenator (10) nach Anspruch 1 oder 2, **dadurch gekennzeichnet, dass** die beiden Lagen (33A, 33B) der Kapillarfasern (32) auch in der zweiten Richtung (Y) versetzt sind.

4. Oxygenator (10) nach einem der vorhergehenden Ansprüche, **dadurch gekennzeichnet, dass** die Berührung zwischen den beiden Lagen (33A, 33B) nur zwischen den Fäden (34A, 34B) jeder Lage (33A, 33B) und den Kapillarfasern (32) der anderen Lage (33A, 33B) erfolgt.

5. Oxygenator (10) nach einem der vorangehenden Ansprüche, **dadurch gekennzeichnet, dass** die Verbindungsgewinde (34A, 34B) einer gleichen Lage (33A, 33B) um eine vorgegebene erste Teilung (P1) beabstandet sind und daß die beiden Lagen (33A, 33B) in der ersten Richtung (X) um einen Abstand versetzt sind, der der Hälfte der ersten Teilung (P1) entspricht.

6. Oxygenator (10) nach Anspruch 3, 4 oder 5, falls von 3 abhängig, **dadurch gekennzeichnet, dass** die Kapillarfasern (32) einer gleichen Lage (33A, 33B) um eine zweite Teilung (P2) beabstandet sind, und dass die beiden Lagen (33A, 33B) in der zweiten Richtung (Y) um einen Abstand versetzt sind, der gleich der Hälfte der zweiten Teilung (P2) ist.

7. Oxygenator (10) nach einem der vorangehenden Ansprüche, **dadurch gekennzeichnet, dass** er eine Kammer (40) für den Eintritt des zu behandelnden organischen Fluids umfasst, die zwischen der dritten Eintrittsöffnung (23) und der Oxygenierungskammer (30) angeordnet ist, und dass die Eintrittskammer (40) statische Verteilungsmittel (41) umfasst.

8. Oxygenator (10) nach Anspruch 7, **dadurch gekennzeichnet, dass** die statischen Verteilungsmittel (41) Flossen mit vorzugsweise einstückig gefertigten Verteilern mit einer oberen Fläche (20A) des Behälterkörpers (20) umfassen.

9. Oxygenator (10) nach Anspruch 8, **dadurch gekennzeichnet, dass** die Flossen (41) eine flache Unterseite (42) aufweisen, und dass die Unterseiten (42) aller Flossen (41) auf derselben Ebene vorgesehen sind.

10. Oxygenator nach einem der vorangehenden Ansprüche, **dadurch gekennzeichnet, daß** er eine Austrittskammer (50) für das zu behandelnde organische Fluid umfasst, die zwischen der Oxygenierungskammer (30) und der vierten Austrittsöffnung (24) angeordnet ist, und dass die Austrittskammer (50) eine zweite statische Ausgangskammer (50) eine zweite statische Verteilungseinrichtung (51) umfasst.

11. Oxygenator (10) nach Anspruch 10, **dadurch gekennzeichnet, dass** die zweiten statischen Verteilermittel (51) mit vorzugsweise einstückig hergestellten Flossen mit einer Unterseite (20B) des Behälterkörpers (20) hergestellt sind.

12. Oxygenator (10) nach Anspruch 11, **dadurch gekennzeichnet, dass** die Flossen (51) eine Oberseite (52) aufweisen, und dass die Oberseiten (52) aller Flossen (51) in der gleichen Ebene vorgesehen sind.

13. Verfahren zum Ausbilden einer Masse (31) von Kappilarfasern (32), die in eine Oxygenierungskammer (30) eines Oxygenators (10) einzusetzen sind, das bereitgestellt ist, um mindestens zwei Lagen (33A, 33B) von Kappilarfasern (32) bereitzustellen, von denen jede eine Vielzahl von Kappilarfasern (32) umfasst, die parallel überlappend in einer ersten Richtung (X) ausgerichtet sind, und mindestens zwei Fäden (34A, 34B), die mit den Kappilarfasern (32) verbunden sind,
wobei die beiden Fäden (34A, 34B) in einer zweiten Richtung (Y) senkrecht zur ersten Richtung (X) ausgerichtet sind und die Lagen (33A, 33B) in Berührung miteinander entlang einer jeweiligen Oberfläche davon anzuordnen, wobei die Lagen (33A, 33B) so vorzusehen sind, dass die Fäden (34A) einer ersten Lage (33A) in Bezug auf die Fäden (34B) der anderen Lage (33B) in der ersten Richtung (X) versetzt sind.

14. Verfahren nach Anspruch 13, **dadurch gekennzeichnet, dass** die Lagen (33A, 33B) von entsprechenden Walzen (330A, 330B) zugeführt werden, die in der ersten Richtung (X) versetzt vorgesehen sind.

15. Verfahren nach Anspruch 13 oder 14, **dadurch gekennzeichnet, dass** vorgesehen ist, die Lagen (33A, 33B) so anzuordnen, dass die gegenseitige Berührung nur zwischen den Fäden (34A, 34B) jeder Lage (33A, 33B) und den Kappilarfasern (32) der anderen Lage (33A, 33B) besteht.

## Revendications

1. Oxygénateur (10) de fluides organiques, comprenant
- un corps de récipient (20) ;
- une première ouverture (21) pour l'entrée d'oxygène et une deuxième ouverture (22) pour la sortie d'un gaz d'évacuation, toutes deux obtenues dans ledit corps de récipient (20) ;
- une troisième ouverture (23) pour l'entrée d'un fluide organique à oxygéner et une quatrième ouverture (24) pour la sortie d'un fluide organique oxygéné, toutes deux obtenues dans ledit corps de récipient (20) ;
- une chambre d'oxygénation (30) pour oxygéner ledit fluide organique à oxygéner, définie à l'intérieur dudit corps de récipient (20) ;
- une masse (31) de fibres capillaires (32) imperméables aux liquides et poreuses aux gaz, disposées de manière à être rodées par ledit fluide organique à l'intérieur de ladite chambre d'oxygénation (30), parallèlement les unes aux autres dans une première direction (X) ;
dans lequel ladite masse (31) de fibres capillaires (32) comprend au moins deux feuilles (33A, 33B) de fibres capillaires (32) connectées l'une à l'autre au moyen d'au moins deux fils de connexion (34A, 34B) qui s'étendent dans une seconde direction (Y) perpendiculaire à ladite première direction (X), lesdites feuilles 25 (33A, 33B) étant en contact réciproque le long d'une surface respective de celles-ci, ledit oxygénateur (10) étant **caractérisé en ce que** lesdites feuilles (33A, 33B) sont disposées de telle sorte que les fils (34A) d'une première feuille (33A) sont décalés par rapport aux fils (34B) de l'autre feuille (33B) dans ladite première direction (X).

2. Oxygénateur (10) selon la revendication 1, **caractérisé en ce que** les deux feuilles (33A, 33B) sont décalées l'une par rapport à l'autre dans la première direction (X).

3. Oxygénateur (10) selon la revendication 1 ou 2, **caractérisé en ce que** les deux feuilles (33A, 33B) de fibres capillaires (32) sont également décalées dans la seconde direction (Y).

4. Oxygénateur (10) selon l'une quelconque des revendications précédentes, **caractérisé en ce que** le contact entre les deux feuilles (33A, 33B) est réalisé uniquement entre les fils (34A, 34B) de chaque feuille (33A, 33B) et les fibres capillaires (32) de l'autre feuille (33A, 33B).

5. Oxygénateur (10) selon l'une quelconque des revendications précédentes, **caractérisé en ce que** les fils de connexion (34A, 34B) d'une même feuille (33A, 33B) sont distants d'un premier pas prédéfini (P1), et **en ce que** les deux feuilles (33A, 33B) sont décalées dans la première direction (X) d'une distance égale à la moitié dudit premier pas (P 1).

6. Oxygénateur (10) selon la revendication 3, 4 ou 5 si dépendante de 3, **caractérisé en ce que** les fibres capillaires (32) d'une même feuille (33A, 33B) sont espacées d'un second pas (P2), et **en ce que** les deux feuilles (33A, 33B) sont décalées dans la seconde direction (Y) d'une distance égale à la moitié dudit second pas (P2).

7. Oxygénateur (10) selon l'une quelconque des revendications précédentes, **caractérisé en ce qu**'il comprend une chambre (40) pour l'entrée du fluide organique à traiter, située entre la troisième ouverture d'entrée (23) et la chambre d'oxygénation (30), et en ce que ladite chambre d'entrée (40) comprend des moyens de distribution statique (41).

8. Oxygénateur (10) selon la revendication 7, **caractérisé en ce que** les moyens de distribution statique (41) comprennent des ailettes réalisées de préférence d'une seule pièce avec une surface supérieure (20A) du corps de récipient (20).

9. Oxygénateur (10) selon la revendication 8, **caractérisé en ce que** les ailettes (41) ont une surface inférieure plate (42), et **en ce que** lesdites surfaces inférieures plates (42) de toutes les ailettes (41) sont disposées sur le même plan.

10. Oxygénateur selon l'une quelconque des revendications précédentes, **caractérisé en ce qu**'il comprend une chambre de sortie (50) du fluide organique à traiter, située entre la chambre d'oxygénation (30) et la quatrième ouverture de sortie (24), et en ce que ladite chambre de sortie (50) comprend des seconds moyens de distribution statique (51).

11. Oxygénateur (10) selon la revendication 10, **caractérisé en ce que** les seconds moyens de distribution statique (51) comprennent des ailettes réalisées de préférence d'une seule pièce avec une surface inférieure (20B) du corps de récipient (20).

12. Oxygénateur (10) selon la revendication 11, **caractérisé en ce que** les ailettes (51) ont une surface supérieure plate (52), et **en ce que** lesdites surfaces supérieures plates (52) de toutes les ailettes (51) sont disposées sur le même plan.

13. Procédé pour former une masse (31) de fibres capillaires (32) à insérer dans une chambre d'oxygénation (30) d'un oxygénateur (10), qui prévoit de mettre à disposition au moins deux feuilles (33A, 33B) de fibres capillaires (32), dont chacune comprend une pluralité de fibres capillaires (32) orientées parallèlement dans une première direction (X), et au moins deux fils (34A, 34B) pour connecter les fibres capillaires (32), lesdits deux fils (34A, 34B) étant orientés dans une seconde direction (Y) perpendiculaire à ladite première direction (X), et de disposer lesdites feuilles (33A, 33B) en contact réciproque le long d'une surface respective de celles-ci, dans lequel lesdites feuilles (33A, 33B) sont disposées de manière à ce que les fils (34A) d'une première feuille (33A) soient décalés par rapport aux fils (34B) de l'autre feuille (33B) dans ladite première direction (X).

14. Procédé selon la revendication 13, **caractérisé en ce que** les feuilles (33A, 33B) sont alimentées par des bobines respectives (330A, 330B) disposées décalées dans la première direction (X).

15. Procédé selon la revendication 13 ou 14, **caractérisé en ce qu**'il est prévu de disposer lesdites feuilles (33A, 33B) de telle sorte que le contact réciproque soit effectué uniquement entre les fils (34A, 34B) de chaque feuille (33A, 33B) et les fibres capillaires (32) de l'autre feuille (33A, 33B).
